# EUROPÄISCHE PATENTSCHRIFT

(11) **EP 0 929 339 B1**
(45) Veröffentlichungstag und Bekanntmachung des Hinweises auf die Patenterteilung: **12.02.2003**
(21) Anmeldenummer: 97954339.4
(22) Anmeldetag: 10.09.1997
(51) Int. Cl.: A61M 25/10

(54) **MAGENSONDE**
STOMACH PROBE
SONDE STOMACALE

(30) Priorität: 10.09.1996 DE 19636654; 23.09.1996 DE 19638935; 23.09.1996 DE 19654910; 07.06.1997 DE 19724096
(43) Veröffentlichungstag der Anmeldung: 21.07.1999
(73) Patentinhaber: Dr. Fred Göbel Patentverwaltung GmbH, 69115 Heidelberg (DE)
(72) Erfinder: Göbel, Fred G., 93047 Regensburg (DE)
(74) Vertreter: Grünecker, Kinkeldey, Stockmair & Schwanhäusser Anwaltssozietät
(86) Internationale Anmeldenummer: EP9704960
(87) Internationale Veröffentlichungsnummer: WO98013090

(56) Entgegenhaltungen:
- FR-A- 2 380 786
- US-A- 3 913 565
- US-A- 4 526 196
- US-A- 4 543 951
- US-A- 5 038 777
- US-A- 5 304 134
- US-A- 5 398 692
- US-A- 5 456 251
- US-A- 5 462 528
- US-A- 5 490 839

## Beschreibung

Die Erfindung betrifft eine Magensonde für beatmete Intensivpatienten.

Aus dem Magen- und Darmtrakt in den Rachen aufsteigendes Material, stellt vor allem bei beatmeten Intensivpatienten ein permanentes Reservoir an Keimen dar, und kann bei der Intubation vom unteren Rachen aus pulmonale Infektion auslösen, bzw. begünstigen. Beatmete Intensivpatienten werden mit sekretfördernden Magensonden bzw. Ernährungssonden versorgt, deren Förderleistung in der Regel jedoch nicht ausreicht, um den gastro-ösophago-pharyngealen Reflux, an der Magensonde vorbei bzw. an ihr entlang zu unterbinden. Als Folge wird die überwiegende Zahl der sedierten und beatmeten Patienten im Bereich von Rachen, Nase und Nasennebenhöhlen von bakteriell hochgradig besiedeltem Material förmlich überschwemmt.

Dieses infektiologische Problempotential konnte bisher aufgrund verschiedenster Schwierigkeiten beim Versuch einer simplen mechanischen, langzeitverträglichen ösophageafen bzw. gastralen Refluxblockade nicht zufriedenstellend gelöst werden. Es wird therapeutisch im wesentlichen medikamentös-antibiotisch (z.B. selektive Darm Dekontamination) angegängen, bzw. überhaupt toleriert.

Da die ösophagealen Wandstrukturen äußerst sensibel auf permanente Druck- bzw. Spannungsexposition reagieren, sind die herkömmlichen Blockungstechniken, bei denen die Wandung der zu verschließenden Struktur (in der Regel durch einen Ballon) unter Spannung genommen wird, in Bezug auf den Ösophagus nicht, oder nur mit Einschränkungen anwendbar.

In der FR-A-2 380 786 ist ein Ballonkatheter zum Aufdehnen von Gefäßen und Hohlorganen offenbart, mit dem Wandgefüge bzw. das retraktive Verhalten von Wandstrukturen dauerhaft verändert werden soll. Eine flexible äußere und eine flexible innere Röhre sind konzentrisch zueinander geordnet, wobei sich zwischen der äußeren Röhre und der inneren Röhre ein äußeres Lumen ausbildet, wobei die innere Röhre ein inneres Lumen bildet. Das äußere Lumen mündet in einen aufblasbaren Ballon, durch welchen sich die innere Röhre hindurcherstreckt. Der Ballon kann über das äußere Lumen mit Hilfe einer Spritze selektiv bis zu einem an einem Manometer ablesbaren bestimmten Druck aufgeblasen werden, um das jeweilige Gefäß bleibend aufzudehnen. Damit sollen z.B. Verengungen von Blutgefäßen oder des Ösophagus aufgedehnt werden, wobei eine bleibende Gewebsveränderung angestrebt ist.

Die US-A-5,304,134 beschreibt einen Ballonkatheter zum bleibenden Aufdehnen von Gefäßen. Zwei konzentrisch angeordnete Röhren bilden zwischen sich ein äußeres Lumen, welches stirnseitig in den Ballon mündet. Das innere Lumen der inneren Röhre erstreckt sich durch den Ballon hindurch. Der Ballon wird hydraulisch unter Druck gesetzt, um ein Gefäß aufzudehnen. Bei der Verwendung zum Einbringen oder Entfernen von Fluiden in den Körper wird der Ballon so stark aufgeweitet bis der Reflux über die Ballonbarriere hin unterbunden ist.

In der US-A-5,462,528 ist eine Magensonde beschrieben, bei der ein aufblasbarer Ballon in den Magenraum vorgeschoben und dort aufgeblasen wird. Über eine visuelle Druckkontrolleinrichtung wird der Ballon soweit aufgeblasen, bis er eine ausreichende Größe erlangt. Ein Einwegventil sichert, dass das Volumen des Ballons stets eine bestimmte Größe hat. Anschließend wird die Magensonde von außen gezogen, bis der Ballon am Eingang der Speiseröhre anliegt. Diese Lage wird durch Festspannen eines Klemmstücks gegen die Nasenöffnung aufrechterhalten.

Bei einem in der US-A-5,490,839 offenbarten Ballonkatheter ist der Ballon doppelwandig ausgebildet, wobei die innere Wandung dem Ballon drucklos eine bestimmte komprimierte Form gibt. Nach dem Einführen in ein Gefäß wird der Ballon soweit aufgeblasen, bis er die entsprechende Wandstrukturen bleibend aufdehnen kann.

Die US-A-5,456,251 offenbart einen Tonometer zum Messen intrakorporaler Drücke, z.B. im Magen oder im Dickdarm. Am Ende eines Schlauches mit mehreren Kanälen ist ein Ballon vorgesehen, der mit einer Flüssigkeit befüllbar ist. Auf dem Schlauch sind innerhalb des Ballons Sensoren zum Abfassen der gewünschten Druckwerte angeordnet. Am Anfang und am Ende des Ballons ist jeweils eine Öffnung zum Befüllen des Ballons vorgesehen. Erst nachdem der Ballon bis zu einem bestimmten Druck aufgeblasen ist, können die gewünschten Werte erfasst werden. Die Ballonhaut ist semipermiabel ausgelegt, damit eindringende Stoffe als Probe gewonnen werden können.

Die US 5,398,692 beschreibt eine Vorrichtung und ein Verfahren zum Ermitteln des mittleren Druckes im linken Herzkammervorhof. Hierfür wird ein Ballon mittels eines Katheters in den Ösophagus eingeführt und zwischen dem linken Herzkammervorhof und der Wirbelsäule so in Anlage an den Herzkammervorhof platziert, dass der Ballon den Druck des linken Herzkammervorhofes erfassen kann. Um ausschließlich diesen Druck und keine anderen thorakalen Drücke zu erfassen, hat der Ballon einen geringeren Durchmesser als der Ösophagus. Die Ballonlänge ist dabei so gewählt, dass ein optimaler Längskontakt zwischen dem linken Herzkammervorhof und dem Ballon besteht, und sich der Ballon nicht zu weit unterhalb des linken Herzkammervorhofes erstreckt, um nicht andere Drücke zu erfassen.

Die US-A-3,913,565 offenbart ein Führungsrohr zum Führen eines Behandlungsinstruments zu einer bestimmten Position in einer Körperhöhlung, insbesondere für Darmbehandlungen. Dabei wird das Führungsrohr mit einer etwa am Frontende angebrachten Cuffmanschette durch den Ösophagus und den Magen vorgeschoben, bis sie im Bereich des Zwölffingerdarms zu liegen kommt. Dort wird sie von außerhalb des Körpers aufgeblasen, bis sie den Eingang zum Dünndarm verschließt. In diesem Zustand wird die Zuleitung unterbrochen, so dass der Druck aufrechterhalten wird. Durch einen inneren Kanal kann dann ein Behandlungsgerät bis in den Darm vorgeschoben werden.

Der Erfindung liegt die Aufgabe zugrunde, eine Magensonde zu schaffen, mit der ein Patient hinsichtlich Infektionen möglichst langzeitverträglich und in der Handhabung einfach zu versorgen ist.

Diese Aufgabe wird mit einer Magensonde mit den Merkmalen des Patentanspruchs 1 gelöst.

Indem die Tamponierblase den Ösophagus verschließt, wird verhindert, dass keimhaltiges Material aus dem Magen- und Darmtrakt an der Magensonde vorbei in den Rachen aufsteigt. Dabei gestattet die siebartige Perforation bei einer über den Ösophagus magenwärts oder rachenwärts gerichteten Kontraktionswelle, beispielsweise beim Schluckakt oder beim Erbrechen des Patienten, eine Volumenverschiebung aus der Tamponierblase in das äußere Lumen der Sonde hinein. In Kombination mit der Ausbildung der Tamponierblase erfolgt ein rascher Volumenaustausch zwischen kontrahierten und bereits wieder bzw. noch erschlafften Sondenabschnitten. Die siebartige Konstruktion des äußeren Lumens ermöglicht eine rasche Umverteilung der Ballonfüllung bereits innerhalb der Tamponierblase und setzt einer sich fortpflanzenden Kontraktionswelle des Ösophagus einen leicht zu überwindenden Widerstand entgegen.

Die von einer erfindungsgemäßen Blase ausgehende Tamponierung des Ösophagus folgt dessen physiologischer Dynamik und gewährleistet so ein simples, sich selbsregulierendes und verträgliches Verschließen oder Ausfüllen der Speiseröhre. Das Aufsteigen hochgradig keimbesiedelten Materials aus dem Magenund Darmbereich in den Mund-, Nasen- und/oder Rachenraum wird durch die Erfindung unterbunden. Gemäß der Erfindung wird der gastro-ösophago-pharyngeale Reflux durch eine simple, sich selbstregulierende und verträgliche mechanische Blockade im Bereich des Ösophagus unterbunden. Die Drainage des Mageninhaltes bleibt dabei im Sinne einer regulären Magen- bzw. Ernährungssonde gewährleistet.

Ulcerationen bzw. Nekrosen der ösophagealen Wandstrukturen als Folge einer Langzeitblockierung, sind durch das weitestgehend druckpassive Verhalten der in der Erfindung beschriebenen Tamponierblase ausgeschlossen.

Unmittelbar benachbarte Strukturen, wie die großen Gefäße, die begleitenden Nerven, die Trachea bzw. Hauptbronchien, die Lunge selbst und nicht zuletzt das Herz (vor allem der linke Herzvorhof) sind im Gegensatz zur herkömmlichen Blockung nicht gefährdet.

Der Innenraum der Tamponierblase kann über einen zwischen dem inneren Lumen und dem äußeren Lumen gebildeten Kanal von einer an den Kanal anschließbaren Befüllvorrichtung mit dem Medium befüllbar sein. Einfache Ausführungsbeispiele für eine solche Befüllvorrichtung sind ein Reservoir oder Ausgleichsgefäß, welche insbesondere außerhalb des Patienten angeordnet sein können. In dem Reservoir oder Ausgleichsgefäß ist ein ausreichender Vorrat des Medium enthalten, um den Innenraum der Tamponierblase zu füllen und außerdem den oben genannten funktionellen Schwankungen von Lumen und Wandtonus des Ösophagus durch weitere Abgabe bzw. Aufnahme des Mediums durch vergrößern und verkleinern der Tamponierblase zu entsprechen.

In diesem Zusammenhang ist es weiterhin als vorteilhaft anzusehen, wenn das Medium aktiv dem Innenraum der Tamponierblase insbesondere über den Kanal zugeführt oder aus dem Innenraum abgeführt wird. Eine solche aktive Zuführung bzw. Abführung kann über eine Pumpe erfolgen, die vorzugsweise zur Aufrechterhaltung des weitestgehend druckpassiven Verhaltens der Tamponierblase geregelt wird.

Um ein Medium zu verwenden, das schnell zuführ- und abführbar ist und gleichzeitig zur Aufrechterhaltung einer angemessenen Temperatur im Ösophagus eine ausreichend hohe Wärmekapazität aufweist, kann das Medium eine Flüssigkeit sein, wie beispielsweise Wasser oder dergleichen. Ein solches Medium ist einfach und schnell pumpbar und gut in seiner Temperatur einstellbar.

Ein weiteres Medium, das sich beispielsweise durch seine Kompressibilität und damit durch eine gewisse Eigenanpaßbarkeit an die oben genannten funktionellen Schwankungen auszeichnet, ist beispielsweise ein gasförmiges Medium. Befüllvorrichtungen, Reservoir, Ausgleichsgefäß und Pumpe sind je nach verwendetem Medium an dieses angepaßt und entsprechend ausgebildet.

Weitere vorteilhafte Ausgestaltungen der Erfindung ergeben sich durch die Merkmale der Unteransprüche.

Die Erfindung wird nachstehend anhand der Figuren an Ausführungsbeispielen näher erläutert:
- Fig. 1: eine Frontalansicht des Ösophagus (Speiseröhre) mit den angrenzenden anatomischen Strukturen, sowie darin eingezeichnet, die im folgenden dargestellte Kombination von Magensonde und Tamponierblase,
- Fig. 2: den Aufbau der Vorrichtung mit Magensonde, Tamponierballon, großlumiger Zuleitung und extrakorporalem Reservoir in einer bevorzugten Ausführungsform der Erfindung,
- Fig. 3: einen Schnitt der Linie I-I der Figur 2;
- Fig. 4: in vereinfachter Darstellung einen Schnitt durch die Wandung einer weiteren möglichen Ausführungsform der Tamponierblase der Magensonde;
- Fig. 5: einen aus einem elastischen Material, beispielsweise aus Gummi hergestellten ballartigen Druckbehälter zur Verwendung bei der Magensonde der Figuren 1 - 3 anstelle des offnenen Reservoirs;
- Fig. 6: in vereinfachter Darstellung eine Magensonde mit einer Druckmeßeinrichtung;
- Fig. 7: in vereinfachter Darstellung ein von dem Signal des Drucksensors abgeleitetes und auf einem Bildschirm dargestelltes Diagramm.

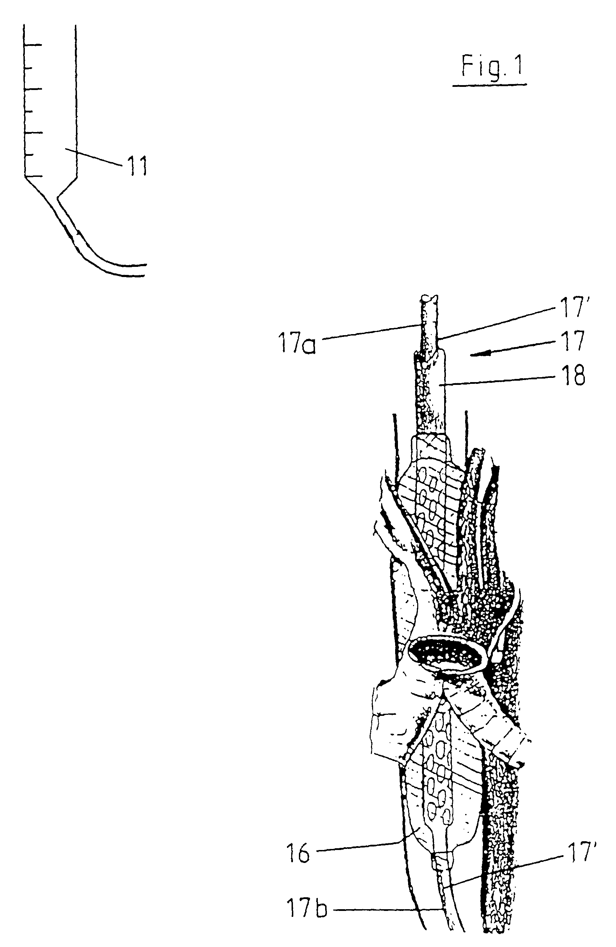

In der Fig. 1 ist die topographische Beziehung der korrekt plazierten Vorrichtung bzw. Magensonde 17, bestehend aus einem schlauchartigen Sondenelement oder Lumen 17' mit Tamponierblase 16, zu den relevanten angrenzenden anatomischen Strukturen (Trachea bzw. Hauptbronchien und große arterielle Gefäße) dargestellt. Wie gezeigt, ist im Bereich des mittleren Ösophagusdrittels eine etwa 15 bis 20 cm lange, mit Wasser (oder einem anderen geeigneten Medium) gefüllte Tamponierblase 16 positioniert. Durch die Taniponierblase 16 hindurch zieht sich die reguläre Ernährungs- bzw. Magensonde bzw. das Lumen 17', die mit ihrem unteren Ende 17b im Magen zu liegen kommt, und mit ihrem oberen Ende 17a oral bzw. nasal ausgeführt wird. Die enterale Ernährung, bzw. die Drainage von Magen- und Darmsekret bleibt auf diese Weise gewährleistet.

Der mit Wasser (oder einem anderen geeigneten Medium) gefüllter Tamponierballon 16 kommuniziert über ein zweites, konzentrisches, äußeres Lumen 18 von möglichst großem Durchmesser mit einem extrakorporalen Reservoir 11. In Fig.1 ist dieser Tamponierballon 16 schraffiert eingezeichnet.

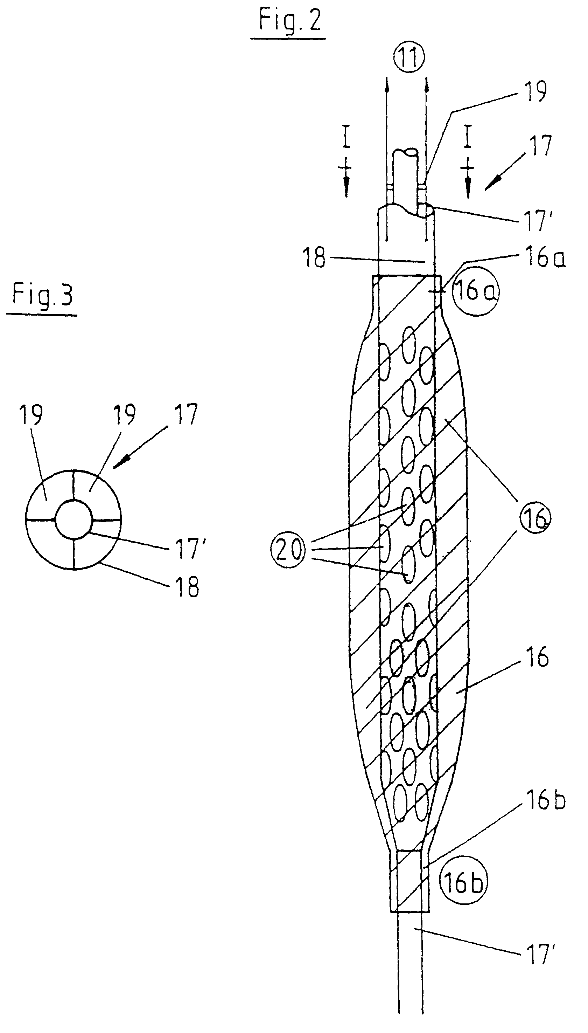

In Fig. 2 ist das Prinzip der gesamten Vorrichtung bestehend aus Sonde oder Sondenschlauch 17' und Tamponierblase 16 näher dargestellt. Die Magensonde 17 ist im Bereich der Tamponierblase 16, sowie in dem von der Tamponierblase zum extracorporal angebrachten Reservoir 11 reichenden Abschnitt der Vorrichtung, von einem konzentrisch angeordneten äußeren Lumen 18 ummantelt. Dieses möglichst groß zu wählende Lumen, gestattet ein rasches Hin- und Herpendeln von Flüssigkeit zwischen der Tamponierblase 16 und Reservoir 11. Nach dem Prinzip kommunizierender Röhren sorgt das äußere Lumen 18 so für eine fortwährende, formerhaltende, sich selbst regulierende Füllung des Tamponierballons 16, ohne dabei die Wand des Ösophagus in nennenswertem Umfang unter Spannung zu nehmen. Die Tamponade der Speiseröhre kann also mit einem minimalen, gerade eben füllungserhaltendenDruck, aufrechterhalten werden. Das äußere Lumen 18 wird dabei durch stegartige Strukturen oder Abstandhalter 19 zwischen der äußeren und inneren Wandung der Sonde aufrechterhalten.

Im vom Tamponierballon 16 umschlossenen Bereich 16a bis 16b, ist die Wandung des äußeren Lumens 18 der konzentrisch-doppeltlumigen Sonde, siebartig perforiert 20. Kommt es beim Schluckakt oder beim Erbrechen zu einer über den Ösophagus (magenwärts oder rachenwärts) gerichteten Kontraktionswelle, gestatten die Perforationen der äußeren Sondenwandung 20 eine Volumenverschiebung aus der Tamponierblase 16 in das äußere Lumen 18 der Sonde hinein, und somit einen raschen Volumenaustausch zwischen kontrahierten und bereits wieder, oder noch erschlafften Sondenabschnitten. Die siebartige Konstruktion des Außenlumens der Sonde ermöglicht also eine rasche Umverteilung der Ballonfüllung bereits innerhalb der Tamponierblase, und setzt so, einer sich beim Schluckvorgang mit einer Geschwindigkeit von etwa 2-3 cm pro Sekunde fortpflanzenden Kontraktionswelle einen leicht zu überwindenden Widerstand entgegen. Größere Volumenverschiebungen, die nicht unmittelbar innerhalb der Tamponierblase ausgeglichen werden können, sind über das Außenlumen der Sonde 18 hinweg, zum extrakorporalen Reservoir 11 hin, möglich. Das Reservoir 11 ist außerhalb und oberhalb des Patienten angebracht und als skalierte Wassersäule gestaltet. Die Sonde 17 bzw. deren Elemente 17', 16 und 18 besteht aus einem körperverträglichen, ausreichend elastischen Material, wie es für herkömmliche Ernährungssonden bereits verwendet wird.

Die Tamponierblase 16 besteht aus einem ebenfalls körperverträglichen, folienartigen, äußerst dehnbaren Material, das ohne Faltenwurf den Form- und Volumenveränderungen des Ösophagus folgt, und sich seinem Epithel anschmiegt. Es ist vorzugsweise eine körperverträgliche Weichfolie, mit latexhaut-ähnlichen Eigenschaften. Die Verbindung zwischen der Tamponierblase 16 und das Lumen 17' bei 16a und 16b erfolgt durch Verkleben, Schrumpfen und Verkleben, Bandagieren oder dergleichen.

Um eine korrekte Lage der Vorrichtung und seiner Tamponierblase im Ösophagus zu gewährleisten, ist die Vorrichtung durch eine farbige Markierung gekennzeichnet, die der späteren Lage im Mundwinkel bei oraler, bzw. an der Nasenöffnung bei nasaler Ausführung entspricht.

Bei Verwendung des Reservoirs 11 ist der Wasserdruck in der Tamponierblase 16 ist allein durch die Wassersäule des bis in das Reservoir 11 anstehenden Wassers bestimmt, wobei sich das Reservoir 11 auf einem Niveau etwas oberhalb der Tamponierblase 16 befindet. Hierdurch wird ein geringfügig über dem Atmosphärendruck liegender, sehr konstanter Innendruck für die Tamponierblase 16 erreicht.

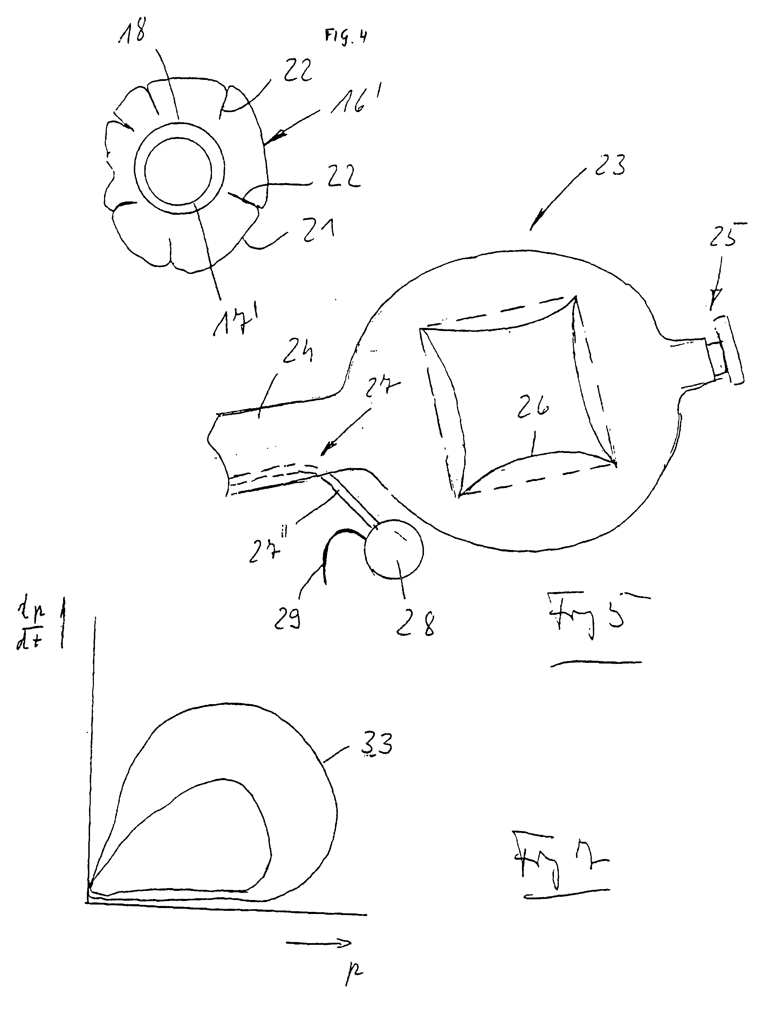

Die Figur 4 zeigt in sehr vereinfachter Darstellung einen Schnitt durch die Tamponierblase 16a bei einer weiteren möglichen Ausführungsform der Erfindung. Die Wandung 21 dieser Tamponierblase besteht aus einem sehr dünnen, flexiblen Material (Folie) und bildet mehrere in das Innere der Tamponierblase 16a hineinstehende Falten 22. An diesen Falten 22 ist beispielsweise das die Wandung 21 bildende Material in geeigneter Weise verklebt oder andersweitig derart verbunden, daß bei einer Beaufschlagung des Innenraumes der Tamponierblase 16a mit dem geringen Druck sich die Falten 22 öffnen und sich somit die Tamponierblase 16a mit ihrer Wandung 22 dicht an die Wand des Ösophagus anlegen kann, und zwar bei unterschiedlichsten anatomischen Gegebenheiten.

Bei dem im Zusammenhang mit den Figuren 1 - 3 beschriebenen System wurde davon ausgegangen, daß der geringfügig über dem Atmosphärendruck liegende Innendruck der Tamponierblase 16 durch die Flüssigkeits- oder Wassersäule mit Hilfe des offenen, extrakorporalen Reservoir 11 eingestellt wird.

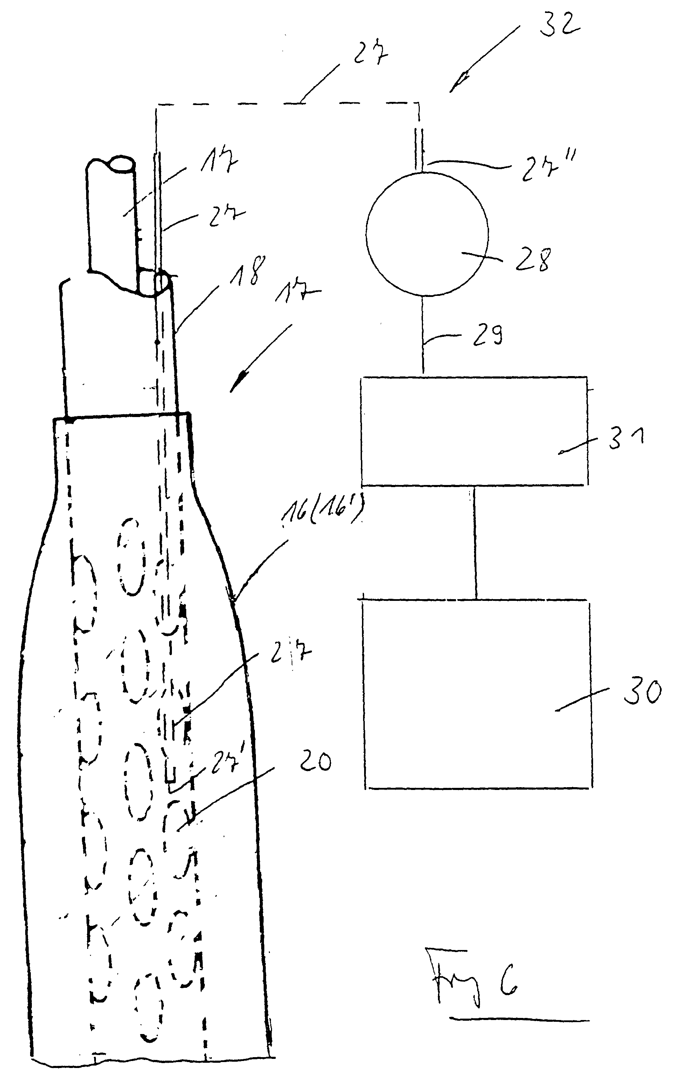

Die Figur 6 zeigt ein geschlossenes Reservoir 23, welches ballartig aus einem elastischen Material, beispielsweise Gummi hergestellt ist. Der Innenraum des Reservoirs 23 ist über den Verbindungsschlauch 24 mit großem Innenquerschnitt mit dem äußeren Lumen 18 der ansonsten nicht weiter dargestellten Magensonde verbunden. Weiterhin besitzt das Reservoir 23 einen stöpselartigen Verschluß 25, über welchem eine Befüllung mit Wasser erfolgen kann, und zwar derart, daß das gesamte System, nämlich die Tamponierblase 16 oder 16', das Lumen 18, der Verbindungsschlauch 24 und das Reservoir 23 vollständig mit dem Wasser ausgefüllt sind. Das Befüllen erfolgt bis zu einem geringen Druck, und zwar derart, daß sich durch die elastischen Eigenschaften der Wandung des Reservoirs 23 der gewünschte geringe Druck einstellt. Zur Einstellung des richtigen Druckes ist auf der Außenfläche der Wandung des Reservoirs 23 eine optische Markierung 26 aufgebracht, und zwar mit einer Linienführung derart, daß dieses Symbol 26 aufgrund der elastischen Dehnung der Wandung des Reservoirs 23 bei dem richtigen Druck eine visuelle leicht erfaßbare symmetrische Figur annimmt. Bei der dargestellten Ausführungsform ist das Symbol 26 ein Viereck, dessen Eckpunkte die Eckpunkte eines Quadrates bilden und dessen Seiten bei drucklosem Reservoir 23 an der Außenseite des Vierecks konkav ausgebildet sind, so daß bei einer Druckbeaufschlagung des Reservoirs 23 diese Markierung 26 die Form eines Quadrates annimmt.

Auch andere Formen sind für das Symbol 26 denkbar, beispielsweise eine ovale, in sich geschlossene Linie, die dann bei dem richtigen Innendruck des Reservoirs 23 in eine Kreisform übergeht usw.

Mit Hilfe des Symbol 26 kann somit der Innendruck auch der Tamponierblase 16 bzw. 16' richtig auf dem geringfügig über dem Atmosphärendruck liegenden Druck eingestellt werden. Das geschlossene System mit dem Reservoir 23 hat den Vorteil, daß dieses Reservoir beispielsweise neben dem Kopf des Patienten auf einer Liege oder einem Bett plaziert werden kann.

Die Figur 6 zeigt in vereinfachter Darstellung nochmals die Magensonde der Figur 2. Mit 27 ist ein dünnes Meßröhrchen bezeichnet, welches mit seinem einen, offenen Ende 27' sich in dem von der Tamponierblase 16 umschlossenen Bereich des äußeren Lumens 18 befindet, und zwar in der Nähe der Öffnungen bzw. Durchbrüche 20. Das Meßröhrchen 27 ist mit seinem anderen Ende aus dem äußeren Lumen 18 und beispielsweise aus dem Verbindungsschlauch 24 herausgeführt, wie dies in der Figur 5 bei 27" dargestellt ist. An diesem Ende 27" befindet sich ein Druckwandler 28, der den Druck innerhalb des Meßröhrchens 27 in ein diesem Druck proportionales elektrisches Signal umwandelt. Der Druckwandler 28 ist über eine Meßleitung 29 an eine u.a. auch einen Bildschirm 30 aufweisende elektronische Einrichtung 31 angeschlossen, die beispielsweise von einem Rechner oder PC gebildet ist. Der Innenraum des Meßröhrchens 27 ist vollständig mit der Flüssigkeit (Wasser) ausgefüllt, so daß der im äußeren Lumen 18 im Bereich der Tamponierblase 16 bzw. 16' wirkende Druck unmittelbar, d.h. nahezu verzögerungsfrei an dem Druckwandler 28 ansteht. Mit dieser Meßeinrichtung lassen sich also intratorakale Drücke oder Druckänderungen problemlos messen, insbesondere auch die Ösophagus-Mobilität, die Respiration und auch die Vorhof- bzw. Kammeraktion des Herzens. Insbesondere läßt sich mit dieser Druckmessung dann auch die Narkosetiefe eines in Narkosetiefe eines in Narkose befindlichen Patienten bestimmen. Das Meßröhrchen 27 und der Druckwandler bilden die Meßsonde 32.

Der mit der Meßsonde 32 bestimmte Druck kann in verschiedenster Weise ausgewertet und/oder auf dem Bildschirm 30 angezeigt werden. Beispielsweise ist eine Anzeige in dem in der Figur 7 wiedergegebenen Diagramm möglich, bei dem es auf der Abszisse der Druck und auf der Ordinate die erste Ableitung des zeitlichen Druckverlaufes aufgetragen sind. Hierdurch ergibt sich eine im sichtbaren Bereich des Bildschirmes verbleibende Kurvenschar von in sich geschlossenen Kurven 33, wobei die Zeitkomponente in dieser Kurvenschar durch unterschiedliche Farbgebung berücksichtigt ist, d.h. durch Umschalten der Farbe in bestimmten Zeitintervallen.

### Bezugszeichenliste

- 11: Reservoir
- 16, 16': Tamponierblase
- 16a, 16b: Ende der Tamponierblase
- 17: Magensonde
- 17', 18: Lumen
- 19: Abstandhalter
- 20: Öffnung
- 21: Wandung
- 22: Falte
- 23: Reservoir
- 24: Verbindungsschlauch
- 25: Verschluß
- 26: Markierung
- 27: Meßröhrchen
- 27', 27": Ende
- 28: Sensor bzw. Druckwandler
- 29: Meßleitung
- 30: Bildschirm
- 31: Meßelektronik
- 32: Meßsonde
- 33: Kurve

## Patentansprüche

1. Magensonde, mit einer im Ösophagus platzierbaren **und den Ösophagus verschließbaren** Tamponierblase (16), welche aus einem flexiblen und/oder elastischen Material wenigstens einen nach außen hin geschlossenen Innenraum zur Aufnahme eines Mediums bildet, mit einem die eigentliche Sonde bildenden inneren Lumen (17'), relativ zu welchem ein bis an die Tamponierblase (16) reichendes äußeres Lumen (18) derart angeordnet ist, dass ein Kanal gebildet ist, der über **Öffnungen** (20) mit dem Innenraum der auf dem äußeren Lumen (18) angeordneten Tamponierblase (16) in Verbindung steht, wobei der Innenraum der Tamponierblase (16) über den Kanal mit einem extrakorporalen Reservoir verbunden ist, welches einen geringen, gerade formerhaltenden Druck in der Tamponierblase gewährleistet, und die Wandung des äußeren Lumens (18) in dem sich längs in der Tamponierblase (16) erstreckenden Bereich mit den den Innenraum der Tamponierblase (16) mit dem Innenraum des äußeren Lumens (18) verbindenden Öffnungen (20) versehen ist, welche über die Länge des äußeren Lumens (18) derart siebartig verteilt angeordnet sind, dass bei Kontraktionswellen des Ösophagus eine rasche Volumenverschiebung des Mediums zwischen kontrahierten und erschlafften Bereichen der Tamponierblase stattfindet.

2. Magensonde nach einem der vorhergehenden Ansprüche,
**dadurch gekennzeichnet,**
**dass** die Tamponierblase (16, 16') Bestandteil eines geschlossenen Flüssigkeitssystems ist.

3. Magensonde nach einem der vorhergehenden Ansprüche,
**dadurch gekennzeichnet,**
**dass** eine Ausgleichsvorrichtung (11) wenigstens das mit der Tamponierblase (16, 16') kommunizierendes Reservoir (23) mit einem Innenraum aufweist, welcher zumindest in einem Teilbereich durch eine flexible oder elastische Wand abgeschlossen ist.

4. Magensonde nach Anspruch 3,
**dadurch gekennzeichnet,**
**dass** das Reservoir ein Mittel zum Einstellen eines vorgegebenen Drucks für das Medium in der Tamponierblase (16) bildet.

5. Magensonde nach wenigstens einem der vorhergehenden Ansprüche,
**dadurch gekennzeichnet,**
**dass** das Medium eine Flüssigkeit ist.

6. Magensonde nach wenigstens einem der Ansprüche 1 bis 4,
**dadurch gekennzeichnet,**
**dass** das Medium gasförmig ist.

7. Magensonde nach einem der vorhergehenden Ansprüche,
**dadurch gekennzeichnet,**
**dass** die Tamponierblase (16) etwa 15 bis 20 cm lang ausgebildet ist.

8. Magensonde nach einem der vorhergehenden Ansprüche,
**dadurch gekennzeichnet,**
**dass** die Tamponierblase derart ausgebildet ist, dass sie mit in das Innere der Tamponierblase (16, 16') hineinstehenden Falten (22) an dem Ösophagus anliegen kann.

9. Magensonde nach Anspruch 8,
**dadurch gekennzeichnet,**
**dass** die Wandung (21) der Tamponierblase (16) an den Falten (22) miteinander verbunden, vorzugsweise verklebt, ist.

10. Magensonde nach einem der vorhergehenden Ansprüche,
**dadurch gekennzeichnet,**
**dass** das äußere Lumen (18) durch stegartige Strukturen oder Abstandshalter (19) relativ zu dem inneren Lumen positioniert ist.

11. Magensonde nach wenigstens einem der vorangehenden Ansprüche,
**dadurch gekennzeichnet,**
**dass** eine Druckmesssonde (32), die mit ihrem Mess-Ende (27') in der Tamponierblase (16, 16') oder in einem mit dem Innenraum der Tamponierblase in Verbindung stehenden Raum, beispielsweise in dem Innenraum des äußeren Lumens (18), platziert ist.

12. Magensonde nach Anspruch 11,
**dadurch gekennzeichnet,**
**dass** die Sonde (32) von einem Messröhrchen (27) gebildet ist, welches mit einem offenen Ende das Mess-Ende bildet und an einem anderen Ende extrakorporal angeordnet einen Drucksensor oder Druckwandler (28) aufweist, und das Messröhrchen (27) mit der Flüssigkeit derart gefüllt ist, dass der gesamte in dem Messröhrchen zwischen den beiden Enden (27, 27') gebildete Kanal mit der Flüssigkeit ausgefüllt ist.

## Claims

1. Stomach probe, comprising a tampon-bladder (16) which is placeable in the esophagus and closable the esophagus and which forms, from a flexible and/or elastic material, at least one inside space, closed to the exterior, for the reception of a medium, an inner lumen (17') forming the actual probe, relative to which an outer lumen (18) reaching to the tampon-bladder (16) is arranged such that a channel is formed which communicates via openings (20) with the inner cavity of the tampon-bladder (16) arranged on the outer lumen (18), the inner cavity of the tampon-bladder (16) being connected via the channel to an extracorporeal reservoir which ensures a small, just shape-maintaining pressure in the tampon-bladder, and the wall of the outer lumen (18) is provided in the area extending longitudinally in the tampon-bladder (16) with openings (20) which connect the inner cavity of the tampon-bladder (16) with the interior of the outer lumen (18) and which are arranged in sieve-like distributed fashion over the length of the outer lumen (18) such that in the case of contraction waves of the esophagus a rapid volume shifting of the medium takes place between contracted and relaxed areas of the tampon-bladder.

2. Stomach probe according to any one of the preceding claims,
**characterized in**
**that** the tampon-bladder (16, 16') is a constituent of a closed liquid system.

3. Stomach probe according to any one of the preceding claims,
**characterized in**
**that** an equalizing device (11) includes at least the reservoir (23) communicating with the tampon-bladder (16, 16') and having an inner cavity which in at least one area is closed off by a flexible or elastic wall.

4. Stomach probe according to claim 3,
**characterized in**
**that** the reservoir forms a means for setting a predetermined pressure for the medium in the tampon-bladder (16).

5. Stomach probe according to at least one of the preceding claims,
**characterized in**
**that** the medium is a liquid.

6. Stomach probe according to at least one of claims 1 to 4,
**characterized in**
**that** the medium is gaseous.

7. Stomach probe according to at least one of the preceding claims,
**characterized in**
**that** the tampon-bladder (16) is designed to be about 15 to 20 cm long.

8. Stomach probe according to any one of the preceding claims,
**characterized in**
**that** the tampon-bladder is designed such that it can rest on the esophagus with folds (22) projecting into the interior of the tampon-bladder (16, 16').

9. Stomach probe according to claim 8,
**characterized in**
**that** the wall (21) of the tampon-bladder (16) is connected to the folds (22), preferably by mutual adhesion.

10. Stomach probe according to any one of the preceding claims,
**characterized in**
**that** the outer lumen (18) is positioned relative to the inner lumen by web-like structures or spacers (19).

11. Stomach probe according to at least one of the preceding claims,
**characterized in**
**that** a pressure-measuring probe (32) is placed with its measuring end (27') in the tampon-bladder (16, 16') or in a space communicating with the inner cavity of the tampon-bladder, for example in the inner cavity of the outer lumen (18).

12. Stomach probe according to claim 11,
**characterized in**
**that** the probe (32) is formed by a small measuring tube (27) which with an open end forms the measuring end and which at another end extracorporeally includes a pressure sensor or pressure transducer (28) and that the small measuring tube (27) is filled with the liquid such that the whole channel formed in the small measuring tube between the two ends (27, 27') is filled with the liquid.

## Revendications

1. Sonde stomacale, comportant une vessie tampon (16) pouvant être placée dans l'oesophage et pouvant obturer l'oesophage, laquelle constitue au moins un espace intérieur en un matériau souple et/ou élastique, fermé vers l'extérieur, pour recevoir un fluide, comportant une lumière intérieure (17') constituant la sonde proprement dite, par rapport à laquelle une lumière extérieure (18) arrivant jusqu'à la vessie tampon (16) est disposée de manière telle qu'un canal est formé, qui par des orifices (20) est en liaison avec l'espace intérieur de la vessie tampon (16) disposée sur la lumière extérieure (18), l'espace intérieur de la vessie tampon (16) étant relié par le canal avec un réservoir extracorporel, lequel assure dans la vessie tampon une pression faible, conservant tout juste la forme, et la paroi de la lumière extérieure (18) dans la zone s'étendant le long de la vessie tampon (16) est pourvue des orifices (20) reliant l'espace intérieur de la vessie tampon (16) avec l'espace intérieur de la lumière extérieure (18), lesquels sont répartis à la manière d'un tamis sur la longueur de la lumière extérieure (18) de manière telle que dans le cas d'ondes de contraction de l'oesophage un déplacement velumique rapide du fluide a lieu, entre les zones contractées et relâchées de la vessie tampon.

2. Sonde stomacale selon l'une des revendications précédentes, **caractérisée en ce que** la vessie tampon (16, 16') est un composant d'un système de liquide fermé.

3. Sonde stomacale selon l'une des revendications précédentes, **caractérisée en ce qu'**un dispositif de compensation (11) présente au moins le réservoir communiquant avec la vessie tampon (16, 16') comportant un espace intérieur, lequel est fermé au moins dans une zone partielle, par une paroi souple ou élastique.

4. Sonde stomacale selon la revendication 3, **caractérisée en ce que** le réservoir constitue un moyen pour régler une pression donnée pour le fluide dans la vessie tampon (16).

5. Sonde stomacale selon l'une des revendications précédentes, **caractérisée en ce que** le fluide est un liquide.

6. Sonde stomacale selon l'une des revendications précédentes, **caractérisés en ce que** le fluide est gazeux.

7. Sonde stomacale selon l'une des revendications précédentes, **caractérisée en ce que** la vessie tampon (16) est réalisée longue d'environ 15 à 20 cm.

8. Sonde stomacale selon l'une des revendications précédentes, **caractérisés en ce que** la vessie tampon est configurée de manière telle qu'elle peut être appliquée sur l'oesophage par des plis (22) pénétrant à l'intérieur de la vessie tampon (16, 16').

9. Sonde stomacale selon la revendication 8, **caractérisée en ce que** la paroi (21) de la vessie tampon (16) est reliée ensemble, de préférence collée, aux plis (22).

10. Sonde stomacale selon l'une des revendications précédentes, **caractérisée en ce que** la lumière extérieure (18) est positionnée par des structures en forme de montants ou entretoises (19) par rapport à la lumière intérieure.

11. Sonde stomacale selon l'une des revendications précédentes, **caractérisée en ce qu'**une sonde de mesure de pression (32) est placés par son extrémité de mesure (27') dans la vessie tampon (16, 16') ou dans un espace en liaison avec l'espace intérieur de la vessie tampon, par exemple dans l'espace intérieur de la lumière extérieure (18).

12. Sonde stomacale selon la revendication 11, **caractérisée en ce que** la sonde (32) est formée par une canule de mesure (27) laquelle par une extrémité ouverte, constitue l'extrémité de mesure et présente à une autre extrémité, disposé en extracorporel, un capteur de pression ou convertisseur de pression (28), et la canule de mesure (27) est remplie de liquide, de manière telle que la totalité du canal formée entre les deux extrémités (27, 27') de la canule de mesure est remplie du liquide,
